# DEMANDE DE BREVET EUROPEEN

(11) **EP 2 742 852 A1**
(43) Date de publication de la demande: **18.06.2014**
(21) Numéro de dépôt: 13193336.8
(22) Date de dépôt: 18.11.2013
(51) Int. Cl.: A61B 3/00, A61B 3/02, A61B 5/16, A61B 5/00

(54) **Système portable et procédé d'exploration du champ visuel d'un individu**

(30) Priorité: 17.12.2012 FR 1262129
(71) Demandeur: JLM Medical, 91140 Villebon Sur Yvette (FR)
(72) Inventeur: LESTRADE, Bernard, 83370 SAINT-AYGULF (FR); PIEDECOCQ, Bertrand, 89120 MARCHAIS BETON (FR); TESTA, André, 56300 PONTIVY (FR)
(74) Mandataire: Pontet Allano & Associes

(57) **Abrégé**

La présente invention concerne un système portable pour explorer le champ visuel d'un patient (202), caractérisé en ce qu'il comprend : une pluralité de sources lumineuses (102₁,..., 102ₙ) disposées à l'intérieur d'un casque (104) porté par le patient (202), les sources lumineuses (102₁,..., 102ₙ) étant agencées de sorte qu'elles sont visibles par le patient (202), des moyens pour commander sélectivement (106) les sources lumineuses (102₁,..., 102ₙ) et des moyens actionnables (108) par le patient (202) pour répondre à des stimuli visuels générés par les sources lumineuses (102₁,..., 102n).

L'invention concerne aussi un procédé mis en oeuvre dans ce système.

## Description

### Domaine technique

La présente invention concerne un dispositif portable pour réaliser une évaluation d'un champ visuel.

Elle concerne également un procédé mis en oeuvre par un tel dispositif.

### Etat de la technique antérieure

On connaît différents modes de réalisation d'un dispositif pour tester les performances visuelles d'un individu par évaluation de son champ visuel.

Les équipements de champ visuel disponibles se divisent en deux catégories : les champs visuels ophtalmologiques et les champs visuels de dépistage. Le prix des équipements de champ visuel ophtalmologique en 2012 pour des champs visuels centraux est de l'ordre de 8600 euros, de l'ordre de 20000 euros pour des champs visuels de type Humphrey Goldman, ce qui limite le nombre d'appareils achetés. De plus, ces équipements ne peuvent être utilisés que par certains spécialistes spécialement formés sur ces appareils. Ils ont l'inconvénient d'être extrêmement lourds, volumineux et totalement intransportable vu leur fragilité et leur poids. Le patient doit se déplacer auprès du champ visuel et non l'inverse. Pour les patients invalides ou alités ces appareils ne peuvent être utilisés.

Les équipements de champ visuel de dépistage actuels, bien moins onéreux, présentent tous les mêmes défauts :
- ils ne testent que le champ visuel temporal et ignorent le champ visuel nasal ;
- le champ visuel temporal n'est testé qu'en trois à quatre points ;
- l'allumage des stimuli se fait systématiquement de l'extérieur vers l'intérieur, ce qui influe sur la prévisibilité du test, qui n'est donc pas aléatoire ;
- les machines restent lourdes et peu mobiles, de l'ordre de 10 à 20 kg ;
- l'encombrement de ces appareils empêche de pouvoir les déplacer facilement, ce qui les rend inutilisables pour par exemple effectuer des contrôles de vision d'un conducteur le long d'une route ou un lit d'un patient ;
- l'absence de caractère aléatoire facilite la tricherie et donc la justesse d'un test de dépistage.

Le but de la présente invention est de proposer un procédé et un dispositif portable d'évaluation de champ visuel d'un patient permettant de pallier au moins un des inconvénients de l'état de l'art rappelés ici :
- proposer un dispositif plus économique que les dispositifs actuels,
- proposer un dispositif utilisable par une personne en général,
- proposer un dispositif testant le champ visuel nasal,
- proposer un dispositif testant le champ visuel temporal sur plus de quatre points,
- proposer un dispositif donc l'allumage des stimuli se fait de manière aléatoire avec la présence de leurres,
- proposer un dispositif léger,
- proposer un dispositif dont l'encombrement permet de le déplacer facilement, en particulier pour effectuer des contrôles de vision d'un conducteur le long d'une route ou au lit d'un patient, et
- proposer un dispositif dont la présence d'un caractère aléatoire rende difficile la tricherie et donc améliore la justesse d'un test de dépistage.

### Exposé de l'invention

Cet objectif est atteint avec un système portable pour explorer le champ visuel d'un patient, **caractérisé en ce qu'il** comprend :
- une pluralité de sources lumineuses disposées à l'intérieur d'un casque porté par ledit patient, lesdites sources lumineuses étant agencées de sorte qu'elles sont visibles par ledit patient,
- des moyens pour commander sélectivement lesdites sources lumineuses, et
- des moyens actionnables par ledit patient pour répondre à des stimuli visuels générés par lesdites sources lumineuses.

De plus, la pluralité de sources lumineuses peut comprendre un premier et un second groupes de sources lumineuses disposées sur un axe sensiblement horizontal pour chaque oeil du patient.

De plus, la pluralité de sources lumineuses peut en outre comprendre un troisième et un quatrième groupes de sources lumineuses disposées par rapport à l'axe sensiblement horizontal avec un angle prédéterminé positif pour chaque oeil du patient.

De plus, la pluralité de sources lumineuses peut en outre comprendre un cinquième et un sixième groupes de sources lumineuses disposées par rapport à l'axe sensiblement horizontal avec un angle prédéterminé négatif pour chaque oeil du patient.

De plus, la pluralité de sources lumineuses peut en outre comprendre des moyens pour visualiser au sein du casque une information sur une demande de test.

De plus, la pluralité de sources lumineuses peut en outre comprendre des moyens pour visualiser au sein du casque une information sur une réponse validée.

Avantageusement, le système portable d'exploration selon l'invention peut en outre comprendre un support sensiblement en arc de cercle solidaire du casque, sur lequel sont disposées tout ou partie des sources lumineuses.

De plus, les moyens de commande sélective peuvent comprendre un microcontrôleur disposé sur le support sensiblement en arc de cercle et connecté d'une part aux sources lumineuses et d'autre part à des moyens de contrôle et de traitement.

De plus, les moyens de réponse peuvent comprendre un poussoir de validation.

De plus, les moyens de commande sélective peuvent être agencés pour commander individuellement tout ou partie des sources lumineuses.

De plus, les moyens de commande sélective peuvent être agencés pour commander l'intensité de toute ou partie des sources lumineuses. Les moyens de commande sélective peuvent être agencés pour commander la durée d'éclairement de ces sources.

Avantageusement, les sources lumineuses peuvent comprendre des diodes électroluminescentes.

De plus, les moyens de visualisation peuvent comprendre des diodes électroluminescentes.

Les avantages de l'utilisation de diodes électroluminescentes comme moyen de stimuli sont nombreux :
- la qualité d'éclairage particulièrement stable (stabilité du spectre en longueur d'onde, stabilité du flux lumineux émis, etc.) ;
- la possibilité d'utiliser une simple liaison USB pour l'alimentation des moyens de stimuli, ce qui permet une alimentation simple par ordinateur ;
- la possibilité de moduler le niveau d'éclairement en fonction des patients et des pathologies.

Les diodes électroluminescentes présentent en outre l'avantage d'être des composants robustes, de faible consommation énergétique

Le système présente une empreinte écologique à la fabrication (carbone) très inférieure aux appareils classiques

Selon un autre aspect de l'invention, il est proposé un procédé pour explorer le champ visuel d'un patient, mis en oeuvre dans un système d'exploration de champ visuel selon l'invention, **caractérisé en ce qu'il** comprend :
- une génération de stimuli lumineux à partir d'une pluralité de sources lumineuses disposées à l'intérieur d'un casque porté par ledit patient, lesdits stimuli lumineux étant commandés sélectivement,
- une réception de réponses dudit patient auxdits stimuli lumineux, et
- un traitement desdites réponses pour produire des informations d'analyse du champ visuel dudit patient.
un dispositif d'évaluation portable d'évaluation de champ visuel d'un patient.

De plus, les commandes sélectives peuvent commander individuellement tout ou partie des sources lumineuses.

### Description des figures et modes de réalisation

D'autres avantages et particularités de l'invention apparaîtront à la lecture de la description détaillée de mises en oeuvre et de modes de réalisation nullement limitatifs, et des dessins annexés suivants :
- la figure 1 représente un mode de réalisation d'un système selon l'invention,
- la figure 2 représente un patient portant un système selon l'invention,
- la figure 3 représente une vue schématique selon une coupe verticale d'un support compris dans un système selon l'invention, et
- la figure 4 représente une vue schématique selon une coupe horizontale d'un support compris dans un système selon l'invention.

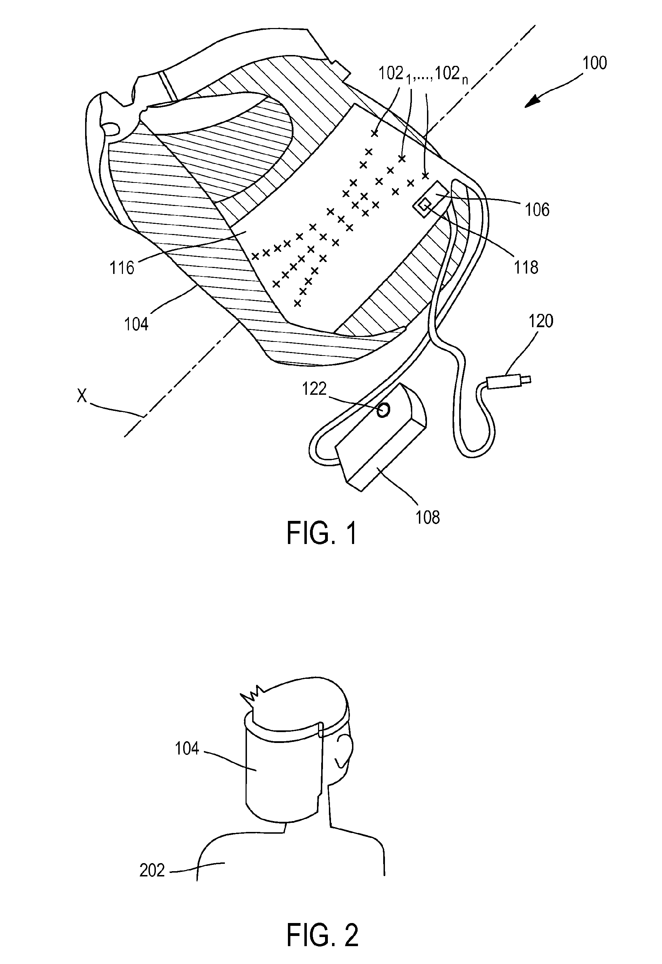

On va maintenant décrire en référence aux figures 1, 2, 3 et 4 un système 100 selon l'invention.

Le système portable 100 permet d'explorer le champ visuel d'un patient 202. Le système portable 100 comprend plusieurs sources lumineuses 102₁,..., 102ₙ. Les sources lumineuses 102₁,..., 102ₙ sont disposées à l'intérieur d'un casque 104.

Le casque 104 est porté par le patient 202. Le profil du casque 104 est choisi pour être le plus enveloppant possible dans le but de s'affranchir des conditions extérieures. Le casque 104 comprend des moyens de réglage dans les trois axes afin de garantir un bon positionnement de la tête.

Les sources lumineuses 102₁,..., 102ₙ sont agencées de sorte qu'elles sont visibles par le patient 202.

Le système portable 100 comprend un support 116. Le support 116 est sensiblement en arc de cercle. Son rayon est d'environ 10 cm. Le support 116 est solidaire du casque 104. Les sources lumineuses 102₁,..., 102ₙ sont disposées sur le support 116. Le support 116 est réalisé en circuit souple.

Le système portable 100 comprend aussi des moyens actionnables 108 par le patient 202 pour répondre à des stimuli visuels générés par les sources lumineuses 102₁,..., 102ₙ. Les moyens de réponse 108 comprennent un poussoir de validation 122.

Les sources lumineuses 102₁,..., 102ₙ comprennent un premier groupe 110₁ et un second groupe 110₂ de sources lumineuses 102₁,..., 102ₙ disposées sur un axe sensiblement horizontal X pour chaque oeil O_{G}, O_{D} du patient 202. Le premier groupe 110₁ et le second groupe 110₂ sont constitués de diodes électroluminescentes d'une couleur jaune disposées respectivement à ±30°, ±40°, ± 60 °, ± 80°, ± 90°, ± 100°, ± 110°, sur l'axe horizontal X pour chaque oeil.

Les sources lumineuses 102₁,..., 102ₙ comprennent un troisième 110₃ et un quatrième groupe 110₄ de sources lumineuses 102₁,..., 102ₙ disposées par rapport à l'axe sensiblement horizontal X avec un angle prédéterminé positif pour chaque oeil O_{G}, O_{D} du patient 202. Les troisième 110₃ et quatrième 110₄ groupes sont également constitués de diodes électroluminescentes de couleur jaune disposées respectivement à ±30°, ±40°, ± 60 °, ± 80°, ± 90°, ± 100°, ± 110°. Les troisième 110₃ et quatrième 110₄ groupes de sources lumineuses 102₁,..., 102ₙ sont disposés selon un plan faisant +30 degrés avec le plan horizontal.

Les sources lumineuses 102₁,..., 102ₙ comprennent un cinquième 110₅ et un sixième 110₆ groupes de sources lumineuses 102₁,..., 102ₙ disposées par rapport à l'axe sensiblement horizontal X avec un angle prédéterminé négatif pour chaque oeil du O_{G}, O_{D} patient 202. Les troisième 110₃ et quatrième 110₄ groupes sont également constitués de diodes électroluminescentes de couleur jaune disposées respectivement à ±30°, ±40°, ± 60 °, ± 80°, ± 90°, ± 100°, ± 110°. Les troisième 110₃ et quatrième 110₄ groupes de sources lumineuses 102₁,..., 102ₙ sont disposés selon un plan faisant -30 degrés avec le plan horizontal.

Les sources lumineuses 102₁,..., 102ₙ comprennent des moyens pour visualiser 112 au sein du casque 104 une information sur une demande de test et des moyens 114 pour visualiser au sein du casque 104 une information sur une réponse validée. Ces moyens de visualisation 112, 114 au sein du casque 104 d'une demande de test et d'une information sur une réponse validée sont mis en oeuvre par deux diodes électroluminescentes bicolores de couleur et rouge bleu disposée à 0° horizontal et vertical et espacées de 8 à 9 centimètres. Lorsqu'elles sont allumées en rouge, une réponse est demandée ou un test est en cours. Lorsqu'elles sont allumées en bleu, une réponse est validée et le poussoir de validation 122 doit être relâché.

Le système portable 100 comprend des moyens pour commander sélectivement 106 les sources lumineuses 102₁,..., 102ₙ.

Les moyens de commande sélective 106 comprennent un microcontrôleur 118. Le microcontrôleur 118 est disposé sur le support 116 sensiblement en arc de cercle. Le microcontrôleur 118 est connecté d'une part aux sources lumineuses 102₁,..., 102ₙ et d'autre part à un ordinateur comprenant des moyens 120 de contrôle et de traitement. Un connecteur USB se trouve sur ce circuit et assure la liaison avec un ordinateur par l'intermédiaire d'un câble de 2,5 mètres situé sur le côté droit du casque. Le poussoir de validation 122 est connecté au câble via une prise jack.

Le système est donc basé sur un circuit unique supportant tous les composants.

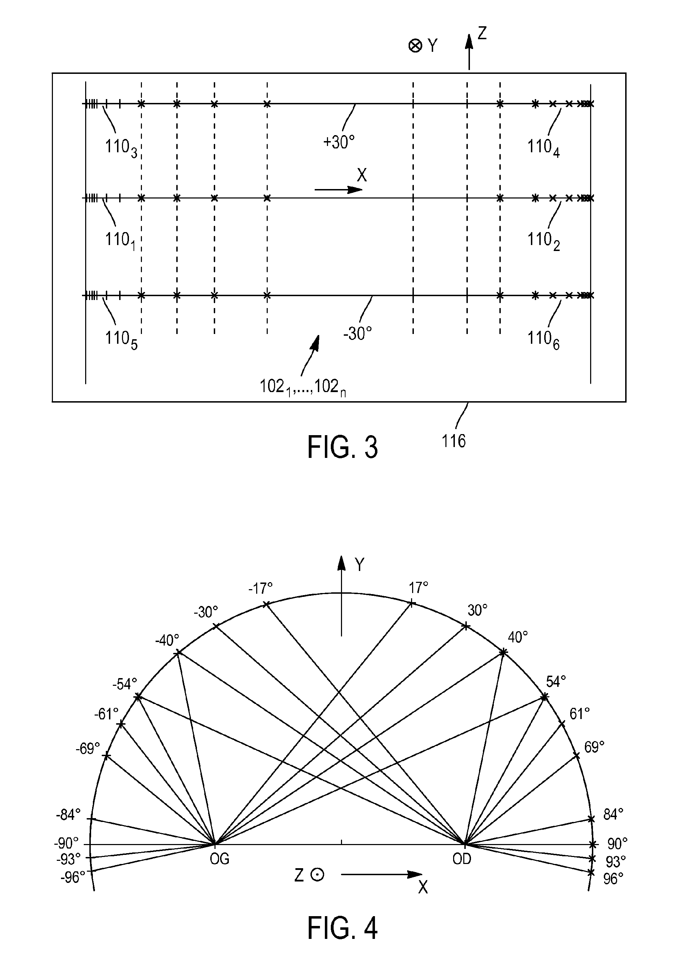

Les figures 3 et 4 représentent plus particulièrement une distribution dans l'espace et sur le support 116 des sources lumineuses 102₁,..., 102ₙ. La figure 4 est une vue schématique de cette distribution selon une coupe horizontale (X,Y) et un axe vertical Z. Des sources lumineuses 102₁,..., 102ₙ sont disposées dans un plan horizontal (X,Y) sur un arc de cercle, en les positions -96°, -93°, -84°, -69°, -61°, -54°, -40°, -30°, -17°, +17°, +30°, +40°, +54°, +61°, +69°, +84°, +90°, +93° et +96° par rapport au centre du cercle. Les sources lumineuses appartenant au premier groupe 110₁ sont représentées par des +. Les sources lumineuses appartenant au second groupe 110₂ sont représentées par des x. On remarque que les sources lumineuses placées en -54, -40, +40, et +54 appartiennent aux deux groupes 110₁ et 110₂ et sont représentées par une superposition des symboles + et x.

La figure 3 est une vue schématique de la distribution de sources lumineuses selon la coupe verticale (X,Z). Les mêmes sources lumineuses sont représentées sur trois axes. L'axe supérieur est l'intersection du plan vertical avec le plan faisant un angle de +30° avec le plan horizontal (X,Y). L'axe milieu est l'intersection du plan vertical avec le plan horizontal. L'axe inférieur est l'intersection du plan vertical avec le plan faisant un angle de - 30° avec le plan horizontal (X,Y).

Les sources lumineuses appartenant au troisième groupe 110₃ sont représentées par des + sur l'axe supérieur. Les sources lumineuses appartenant au quatrième groupe 110₄ sont représentées par des x sur l'axe supérieur. On remarque que les sources lumineuses placées en -54, -40, +40, et +54 appartiennent aux deux groupes 110₃ et 110₄ et sont représentées par une superposition des symboles + et x.

Les sources lumineuses appartenant au cinquième groupe 110₅ sont représentées par des + sur l'axe inférieur. Les sources lumineuses appartenant au sixième groupe 110₆ sont représentées par des x sur l'axe inférieur. On remarque que les sources lumineuses placées en -54, -40, +40, et +54 appartiennent aux deux groupes 110₅ et 110₆ et sont représentées par une superposition des symboles + et x.

Dans un autre mode de réalisation du dispositif selon l'invention, la prise jack est remplacée en faveur d'une connexion renforcée. La connexion est renforcée pour tenir compte de l'usage brutal de certains patients et examinateurs.

Bien sûr, l'invention n'est pas limitée aux exemples qui viennent d'être décrits et de nombreux aménagements peuvent être apportés à ces exemples sans sortir du cadre de l'invention.

En particulier, on peut prévoir autant de diodes électroluminescentes que souhaitées, pour augmenter la précision d'un tracé du champ visuel d'un patient.

## Revendications

1. Système portable (100) pour explorer le champ visuel d'un patient (202), **caractérisé en ce qu'il** comprend :
- une pluralité de sources lumineuses (102₁,..., 102ₙ) disposées à l'intérieur d'un casque (104) porté par ledit patient (202), lesdites sources lumineuses (102₁,..., 102ₙ) étant agencées de sorte qu'elles sont visibles par ledit patient (202),
- des moyens pour commander sélectivement (106) lesdites sources lumineuses (102₁,..., 102ₙ), et
- des moyens actionnables (108) par ledit patient (202) pour répondre à des stimuli visuels générés par lesdites sources lumineuses (102₁,..., 102ₙ).

2. Système portable d'exploration (100) selon la revendication 1, **caractérisé en ce que** la pluralité de sources lumineuses (102₁,..., 102ₙ) comprend un premier (110₁) et un second (110₂) groupes de sources lumineuses (102₁,..., 102ₙ) disposées sur un axe sensiblement horizontal (X) pour chaque oeil (O_{G}, O_{D}) du patient (202).

3. Système portable d'exploration (100) selon la revendication 2, **caractérisé en ce que** la pluralité de sources lumineuses (102₁,..., 102ₙ) comprend en outre un troisième (110₃) et un quatrième (110₄) groupes de sources lumineuses disposées par rapport à l'axe sensiblement horizontal (X) avec un angle prédéterminé positif pour chaque oeil (O_{G}, O_{D}) du patient (202).

4. Système portable d'exploration (100) selon l'une des revendications 2 ou 3, **caractérisé en ce que** la pluralité de sources lumineuses (102₁,..., 102ₙ) comprend en outre un cinquième (110₅) et un sixième (110₆) groupes de sources lumineuses (102₁,..., 102ₙ) disposées par rapport à l'axe sensiblement horizontal (X) avec un angle prédéterminé négatif pour chaque oeil (O_{G}, O_{D}) du patient (202).

5. Système portable d'exploration (100) selon l'une des revendications 2 à 4, **caractérisé en ce que** la pluralité de sources lumineuses (102₁,..., 102ₙ) comprend en outre des moyens pour visualiser (112) au sein du casque (104) une information sur une demande de test.

6. Système portable d'exploration (100) selon la revendication 5, **caractérisé en ce que** la pluralité de sources lumineuses (102₁,..., 102ₙ) comprend en outre des moyens (114) pour visualiser au sein du casque (104) une information sur une réponse validée (108).

7. Système portable d'exploration (100) selon l'une quelconque des revendications précédentes, **caractérisé en ce qu'il** comprend en outre un support (116) sensiblement en arc de cercle solidaire du casque (104), sur lequel sont disposées tout ou partie des sources lumineuses (102₁,..., 102ₙ).

8. Système portable d'exploration (100) selon la revendication 7, **caractérisé en ce que** les moyens de commande sélective (106) comprennent un microcontrôleur (118) disposé sur le support (116) sensiblement en arc de cercle et connecté d'une part auxdites sources lumineuses (102₁,..., 102ₙ) et d'autre part à des moyens (120) de contrôle et de traitement.

9. Système portable d'exploration (100) selon l'une quelconque des revendications précédentes, **caractérisé en ce que** les moyens de réponse (108) comprennent un poussoir de validation (122).

10. Système portable d'exploration (100) selon l'une quelconque des revendications précédentes, **caractérisé en ce que** les moyens de commande sélective (106) sont agencés pour commander individuellement tout ou partie des sources lumineuses (102₁,..., 102ₙ).

11. Système portable d'exploration (100) selon la revendication 10, **caractérisé en ce que** les moyens de commande sélective (106) sont agencés pour commander l'intensité de toute ou partie des sources lumineuses (102₁,..., 102ₙ).

12. Système portable d'exploration (100) selon l'une quelconque des revendications précédentes, **caractérisé en ce que** les sources lumineuses (102₁,..., 102ₙ) comprennent des diodes électroluminescentes.

13. Système portable d'exploration (100) selon l'une des revendications 5 ou 6, **caractérisé en ce que** les moyens de visualisation (112 ; 114) comprennent des diodes électroluminescentes.

14. Procédé pour explorer le champ visuel d'un patient (202), mis en oeuvre dans le système d'exploration de champ visuel selon l'une quelconque des revendications précédentes, **caractérisé en ce qu'il** comprend :
- une génération de stimuli lumineux à partir d'une pluralité de sources lumineuses (102₁,..., 102ₙ) disposées à l'intérieur d'un casque (104) porté par ledit patient (202), lesdits stimuli lumineux étant commandés sélectivement (106),
- une réception de réponses dudit patient (202) auxdits stimuli lumineux, et
- un traitement desdites réponses pour produire des informations d'analyse du champ visuel dudit patient (202).

15. Procédé pour explorer le champ visuel d'un patient (202) selon la revendication précédente, **caractérisé en ce que** lesdites commandes sélectives (106) commandent individuellement tout ou partie des sources lumineuses (102₁,..., 102ₙ).
